# EUROPEAN PATENT SPECIFICATION

(11) **EP 2 928 289 B1**
(45) Date of publication and mention of the grant of the patent: **25.09.2019**
(21) Application number: 13791760.5
(22) Date of filing: 31.10.2013
(51) Int. Cl.: A01M 99/00, A01G 13/10, A01K 67/033

(54) **SYSTEM FOR PROVIDING BENEFICIAL INSECTS OR MITES**
SYSTEM ZUR BEREITSTELLUNG VON NUTZINSEKTEN ODER MILBEN
SYSTÈME DE DIFFUSION D'INSECTES OU D'ACARIENS UTILES

(30) Priority: 05.12.2012 GB 201221900
(43) Date of publication of application: 14.10.2015
(73) Proprietor: BIOLINE AGROSCIENCES LIMITED, Holland Road Little Clacton Essex CO16 9QG (GB)
(72) Inventor: BAXTER, Ian, 3605 MA Maarssen (NL); STEPMAN, Ward, 3605 MA Maarssen (NL); EEKHOFF, Dennis, 3605 MA Maarssen (NL); VAN DIEMEN, Bas, 3605 MA Maarssen (NL); WALKER, Phil, 3605 MA Maarssen (NL)
(74) Representative: Regimbeau
(86) International application number: PCT/EP2013/072780
(87) International publication number: WO 2014/086536

(56) References cited:
- EP-A1- 0 234 404
- US-A1- 2005 178 337

## Description

The present invention relates to a system, method and use in the field of pest control. It relates to the use of beneficial insects or mites and to a system of dispensing said beneficial insects or mites.

Plant pests in the field and in a protected cropping environment cause significant damage to crops and reduce yield. The loss of crops in a commercial plant growing environment is undesirable and expensive, adding to the cost passed on to wholesale buyers and consumers. In addition there is often the additional cost of removing damaged crops from the growing environment.

Insect and mite pests can be controlled by chemical pesticides which although often effective are undesirable for reasons of chemical residue left on the crops or produce which is undesirable for consumers. In addition the development of insects resistant to chemical measures can building up with prolonged use and cause further problems for mite eradication or crop treatment with the resistant strain of pests.

It is known to provide an alternative method of pest control to chemical pesticides using beneficial insects and mites. One example is given in GB2393890 and describes the use of an integrated crop and pest management program. US 2005/0178337 A1 and EP 0 234 404 A1 disclose also systems for providing beneficial insects.

Existing systems provide a predatory mite predatory towards the particular problem pest insect, for example thrips. The provision of the mites is in the manner of a controlled release, manually over a time scale such as a series of days or sprinkled onto crops or provided in sachets and released in a controlled manner over a longer period of time, for example over a growing and cropping period.

Existing sachet systems of one type are described in GB2393890 however these are size and weight limited and fitting the system and implementing it by hand can be labour intensive. Sachets are provided with a hook for individual placement of the sachets onto plants or onto wires above the growing plants, however the hooks may become wet with watering and fail, the sachet may fall to the ground wasting the possibility of the mites being applied to and reaching the crops. The placement is static and is not varied with the growth of the plants and also lacks scalability.

The present invention describes a new system for providing beneficial insects or mites in a protected cropping environment.

According to a first aspect of the present invention there is provided a system according to claim 1 suitable for providing beneficial insects or mites in a protected cropping environment, comprising a carrier ribbon and a plurality of substantially planar sachets carried on the ribbon, wherein each sachet comprises a dispensing compartment for beneficial insects or mites and a first emergence hole on one side of the compartment and a second emergence hole so that insects or mites can emerge and be dispensed in the protected cropping environment. The provision of the sachets on the strip of ribbon allows the beneficial mites to be delivered over a larger area than is customary and with precision.

According to the invention, the sachets are substantially planar. Each sachet may comprise at least two strips of material, one overlaid on the other and sealed together at their edges to form the substantially planar sachet. The planarity of the mite-containing sachets allows the sachets to pack compactly against the carrier ribbon and each other for transport and storage before distributing in the protected cropping environment. The ability of the planar sachets to fold flat against the carrier ribbon also avoids damage to the sachet, carrier ribbon and/or plants in the protected cropping environment.

In one embodiment of the present invention the sachets are fixedly attached to the carrier ribbon at a predetermined location. The sachets are arranged to depend from the carrier ribbon in an embodiment. The arrangement of the sachets provides flexibility in the disbursement of the beneficial mites, whilst retaining the structural integrity of the system and avoiding water damage and wear on the sachets which may occur should they be located or hung directly from a plant or in the crop.

The sachets are arranged along the carrier ribbon in a regularly spaced arrangement and can be arranged in an equally spaced arrangement. The precise location, spacing and arrangement of the sachets can be set as required by the system and the cropping environment and crop spacing to optimise the performance and distribution of the mites. According to the invention, the sachet spacing is in the range from 10 cm to 90 cm, and may be spaced by 75 cm. One embodiment may feature a regularly repeating pattern of sachet spacing. In the system of an embodiment of the invention the sachets are fixed to the carrier ribbon by one of the range of; gluing, welding, stitching, heat treatment, stapling. This provides for a variety of fixing techniques that can be adapted and fine-tuned to suit the material of the sachets and the carrier ribbon.

Preferably, there is at least one emergence hole located on each sachet in an uppermost portion of the compartment adjacent the carrier ribbon. According to the invention, the sachet comprises a first emergence hole and a second emergence hole. Preferably, the emergence hole or holes are located at a distance in the range of 5 to 30 mm from the carrier ribbon. Locating the emergence hole high up on the sachet in the portion nearest the carrier ribbon has been found to ease the exit of insects or mites from the sachet and improve the output count of insects or mites from each sachet.

In one embodiment but not according to the invention, the second emergence hole is located on the same side of the compartment as the first emergence hole and at a position corresponding to the mirror image of the first emergence hole, wherein the mirror line comprises a line bisecting the sachet in a vertical direction from an upper portion to a lower portion of the sachet. According to the present invention the position of the second emergence hole mirrors the position of the first emergence hole on an opposite side face of the sachet.

This configuration of emergence holes where the location of the second emergence hole is a mirror image of the first emergence hole has been found to improve the output of insects or mites from the sachet in instances where the crop grown in the protected cropping environment is one that thrives in high humidity, for example, cucumber.

In one embodiment it can be useful to provide a variety of different types of mites, or an alternating pattern of mites to combat and address a particular pest or mixture of pests and a first sachet on the carrier ribbon provides a first type of beneficial insect or mite and an adjacent sachet provides a second type of beneficial insect or mite. In this way an embodiment of the system can include a first sachet on the carrier ribbon providing a beneficial insect or mite in a first format and an adjacent sachet provides the said beneficial insect or mite in a second format.

The carrier ribbon of an embodiment comprises one material chosen from the range; webbing, woven material, paper, polymer, biodegradable. The material can be water resistant and hard wearing to survive and be used through a cropping cycle and, if required, used again. A suitable material could be waterproof and could be lightweight for ease of handling and manipulation within the cropping environment. In an embodiment the sachet comprises one material chosen from the range; fabric, PE coated paper, strip material. In this way a material is chosen for its suitability to the cropping environment. A carrier ribbon according to the invention is of a length in the range 10m to 200m. The range of 80 to 90m in length is also described in an embodiment. A long length of ribbon is appropriate for use with a length of crop or a line of planting. An appropriate length of ribbon is chosen for a particular, for example, greenhouse or polytunnel. The ribbon should not hinder the crop growth and development and should be unobtrusive and easy to use, maintain and work with. In one embodiment the carrier ribbon is light transmittant, and comprises a material having the property of allowing light to be transmitted through it. In this way there is no restriction in the light reaching the crop and the beneficial mites when they are on the crop, thus aiding and not hampering growth.

A drum in an embodiment carries and stores the carrier ribbon and sachets in a rolled arrangement, this helps to reduce damage and wear to the carrier ribbon and sachets prior to being put to use and provides a means of efficiently storing the system when not in use. The roll also allows each whole turn of the carrier ribbon about the drum to protect the beneficial mites in the sachets and assists with maintaining them in the correct pre-deployment environment and stat, for example by moderating temperature changes and fluctuations. In effect, each sachet in the roll is overlapped and wrapped in an additional layer of carrier ribbon.

According to an embodiment of the present invention there is provided a system suitable for providing beneficial insects or mites in a protected cropping environment, comprising a carrier ribbon and a plurality of sachets carried on the ribbon, a casing for the ribbon, the casing having a front face and an opening in the front face for deploying the ribbon, wherein each sachet comprises a dispensing compartment for beneficial insects or mites and at least one emergence hole on one side of the compartment so that insects or mites can emerge and be dispensed in the protected cropping environment, the carrier ribbon being arranged as a roll, accommodated within the casing and with a first end of the roll accessible at the opening. The provision of a casing and a drum in the embodiment assists with storage and warehousing, for example allowing the casing holding the systems to be stacked.

In one embodiment a plurality of lines of crops are envisaged and a plurality of carrier ribbons, five, are housed in the casing so that each line of crop may be addressed by a different ribbon. In this example each carrier ribbon is dispensed for a different line and each ribbon of sachets can be optimised and tailored to the crops in one particular line.

A casing for one embodiment is a hexagonal container having a top face, a bottom face and six side faces, one side face including an opening through which the carrier ribbon is accessible and can be deployed. The hexagonal structure is space saving and efficient in storage and can be arranged to stand on one of its six side faces for ease of access to the opening through which the carrier ribbon is accessed deployed. In one embodiment there is a means for attaching the carrier ribbon to a mobile station suitable for watering or crop protection spraying for rapid deployment. The mobile station can be moved around and used, as a secondary function to deploy and set out the carrier ribbon and sachets over the crop to be treated.

According to a second aspect of the present invention there is provided a method according to claim 14 of providing beneficial insects or mites in a protected cropping environment, with a system as set out above. In addition the use according to claim 15 of such a system is a further aspect of the present invention.

The term protected cropping environment is designed to be used to describe a variety of indoor growing spaces such as polytunnels, sheds, greenhouses, glass houses, cold frames, plastic tunnels or cloches. The system of the invention could also be used in an outdoor scenario such as a field.

The term ribbon refers to a strip of material and may be any shape or size. Typically the strip will be rectangular and will have sufficient length to accommodate at least one sachet.

The term sachet is one known in the art. A sachet comprises a compartment having a volume that can be filled or part filled with a beneficial mite and food source. An emergence hole is provided on one side of the compartment in order that the beneficial insects or mites can emerge. It is envisaged that there may be more than one compartment and that each compartment, if required, may have more than species of insect or mite. One method of sachet production is described in GB 2393890.

The invention will now be described in more detail, by way of example only, with reference to the accompanying drawings, in which:
Figure 1a is a front view of a sachet to be used with a system but not according to the present invention;
Figure 1b is a front view of a sachet to be used with a system but not according to the present invention, according to an alternative embodiment;
Figure 2 is a perspective view of the system of the present invention in a first configuration;
Figure 3a is a perspective view of an embodiment of the system of the present invention;
Figure 3b is a partial view of Figure 3a;
Figure 4 is a perspective view of an alternative embodiment of the system of the present invention; and
Figure 5 is a front view of a sachet to be used with a system but not according to the present invention, according to an alternative embodiment.

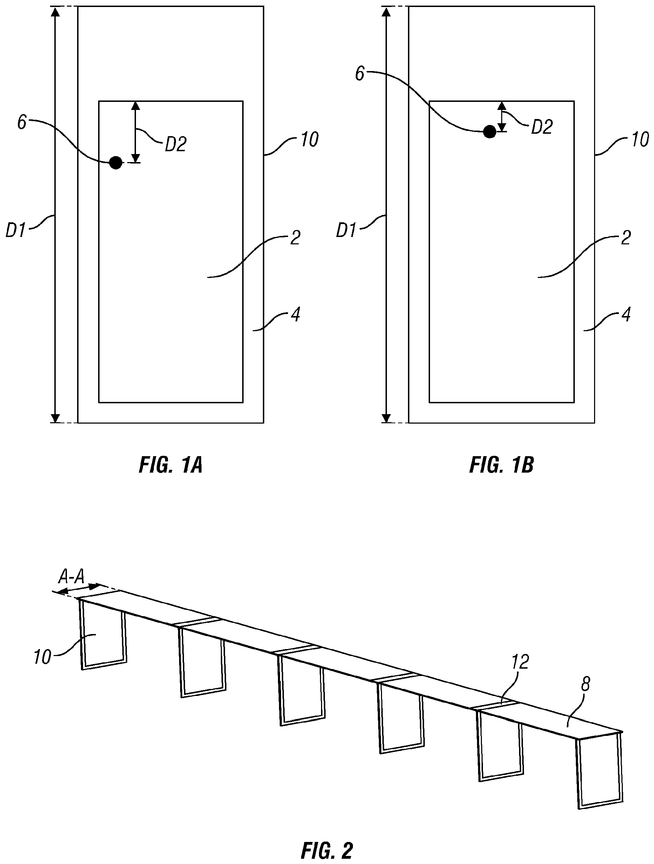

The system suitable for providing beneficial insects or mites in a protected cropping environment will now be described with reference to Figures 1a, 1b and 2.

The system comprises a generally rectangular shaped sachet 10. The sachet 10 comprises a compartment 2, bounded by a sealed border 4. The sachet commonly comprises at least two strips of material of approximately the same size one overlaid on the other, sealed together at their edges.

Beneficial insects or mites are accommodated within the sachet 10.

An emergence hole 6 is provided in the sachet 10 in order that the beneficial mites can exit and disperse from the sachet.

The dimensions of the sachet 10 of the embodiment are width 50mm and length D1 of 160 mm. The volume of the sachet compartment 2 is 40ml, with a mite volume of around 20ml. In other scenarios the size of the sachet 10, from width 40 to 70 mm and length D1 from 80 to 120 mm, and the volume of the compartment 4 may alter, for example from 5 to 40 ml. The width of the sachet and the system may vary depending on the plant rows and spacing in the cropping environment. In Figure 1a, the emergence hole 6 is located in the top left portion of the sachet 10 and the emergence hole 6 is a distance D2 from the sealed border 4 adjacent the carrier ribbon 8. In the embodiment illustrated in Figure 1a D2 is 23mm. In other scenarios, the distance D2 of the emergence hole 6 from the sealed border 4 adjacent the carrier ribbon 8 may alter, for example from 5 to 30 mm. In Figure 1b the emergence hole 6 is located in the top central portion of the sachet 10 at a distance D2 of 5 mm away from the sealed border 4 adjacent the carrier ribbon 8.

The system further comprises a carrier ribbon 8 having a width (A-A) equal or approximately equal to that of the width of the sachets 10. A backing web may be provided. A plurality of sachets 10 are attached at fixing points 12 by bonding, gluing or other means to the carrier ribbon 8. With reference to Figure 2 in the deployed system the sachets hang down from and depend from the carrier ribbon 8.

The fixing points 12 are such that they are stronger and thicker than the ribbon 8 and thus provide greater rigidity and structural support than the ribbon 8 alone.

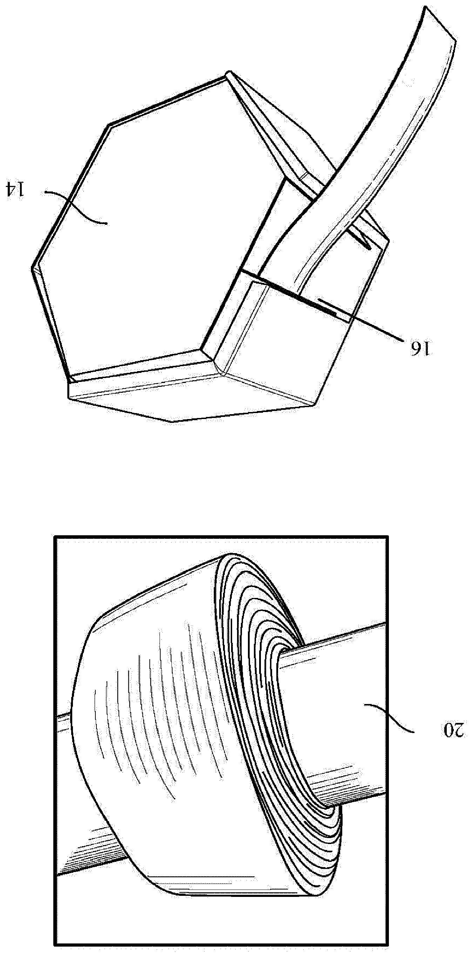

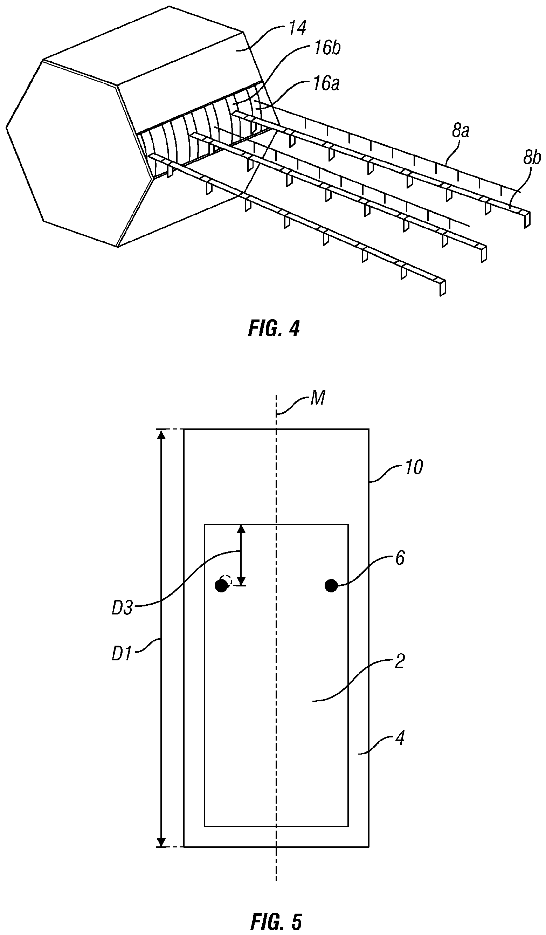

A further embodiment of the present invention will now be described with reference to Figures 3a and 3b and the casing 14. For storage and deployment the carrier ribbon 8 is formed into a roll and loaded onto a drum or spindle 20. The ribbon 8 is accommodated within the casing 14 and the casing 14 comprises hexagonal container having a top face, a bottom face and six side faces, one side face including an opening 16 through which the carrier ribbon 8 is accessible and can be deployed. In an alternative embodiment, illustrated in Figure 4, there are a plurality of lines of carrier ribbon 8 and sachets 10. Five lines are illustrated in Figure 4, each line (8a, 8b ...) emerges from its own opening (16a, 16b, ..) in a side face of the container and casing 14.

Multiple lines are provided to treat crops and disperse across a wide cropping environment. The rolled arrangement provides ventilation for the sachets 10.

The totality of the multiple lines may be of an identical shape, size and orientation format or one or more of the lines may be different. In Figure 4 there are 3 lines shown having the same or identical format and 2 lines are shown schematically having a different format.

In use, the carrier ribbon 8 is accessed from the opening and distributed across the cropping environment. It can be attached to a watering device or other automatic means for distribution.

In an alternative embodiment, illustrated in Figure 5, there are two emergence holes 6 on each sachet 10. The emergence holes 6 are located on the same side of the compartment in the uppermost portion of the compartment at a distance D3 from the sealed border 4 adjacent the carrier ribbon. In the embodiment illustrated in Figure 5 D3 is 23mm. In other scenarios, the distance of the emergence hole from the sealed border 4 adjacent the carrier ribbon 8 may alter, for example a distance between 5 to 30 mm. The location of the second emergence hole is a mirror image of the first emergence hole, wherein the mirror line M comprises a line bisecting the sachet in a vertical direction. In another embodiment (shown in dashed lines), the location of the second emergence hole is a mirror image of the first emergence hole on the opposite side face of the sachet.

A working example of a sachet system is with the predatory mite *Amblyseius swirskii* (Athias-Henriot). Approximately 250 predators are present in a sachet and are sustained on a factitious prey mite, *Suidasia medanensis* Oudemans, which itself, is feeding and breeding on a matrix based on bran and yeast powder. The matrix and live mite mixture is ca. 20ml in volume, with the total volume of the sachet being ca.40ml. This particular format of predatory mite is typically used to control whitefly and thrips infestations, in commercial crops such as cucumber, sweet peppers and ornamental plants such as chrysanthemum. Under typical growing conditions the predatory mites will feed and breed on the factitious prey mite and will be gradually inoculated into the crop over a 3-6 week period, depending on local conditions. With suitable conditions for growth, breeding and sustainment as many as four times the original number of predatory mites placed into the sachet may eventually exit the sachet through the emergence and be inoculated into the crop.

Various modifications may be made to the described embodiments without departing from the scope of the present invention as defined by the appended claims. There may be a different number of ribbons or sachets. The portions or sachet compartments may be altered or be arranged differently. Other options for mites or combination of mites may be used, the same mite in the system or one or more mites could be considered, they may alternate along the strip, in the same sachet or deployed in different sachets along a ribbon. This means that a different treatment plan could be devised with different beneficial mites emerging at different times in a cropping cycle or in a pest control cycle. The size and shape of the drum and casing may be altered as well as the deployment method. The materials of the construction may be altered or changed. Other alternatives for material choices may be required in harsh conditions or for aggressive pests. There may be more than one ribbon or more than one casing used and any number of supporting means may be envisaged.

Alternative forms of construction of the sachet may be considered, such as a circular or square shape. Versions could include regions of the ribbon devoid of sachets for a particular cropping environment as required.

## Claims

1. A system suitable for providing beneficial insects or mites in a protected cropping environment, comprising a carrier ribbon (8) and a plurality of substantially planar sachets (10) carried on the ribbon (8), wherein each sachet (10) comprises a dispensing compartment (2) for beneficial insects or mites and a first emergence hole (6) on one side of the compartment and a second emergence hole (6) so that insects or mites can emerge and be dispensed in the protected cropping environment, wherein the position of the second emergence hole (6) mirrors the position of the first emergence hole (6) on an opposite side face of the sachet (10), and wherein the sachets (10) are arranged along the carrier ribbon (8) in a regularly spaced arrangement said spacing being in the range from 10cm to 90cm; and wherein the carrier ribbon (8) is of a length in the range of 10m to 200m.

2. A system according to claim 1, wherein the sachets (10) are arranged to depend from the carrier ribbon (8), and wherein preferably the sachets are arranged in an equally spaced arrangement.

3. A system according to claim 1 or 2, wherein the sachets (10) are fixed to the carrier ribbon (8) by one of the range of; gluing, welding, stitching, heat treatment, stapling.

4. A system according to any of claims 1 to 3, wherein the first emergence hole (6) and second emergence hole (6) are located in an uppermost portion of the compartment adjacent the carrier ribbon (8), and wherein preferably, the emergence holes (6) are located at a distance in the range of 5 to 30 mm from the carrier ribbon (8).

5. A system according to any preceding claim, wherein a first sachet (10) on the carrier ribbon (8) provides a first type of beneficial insect or mite and an adjacent sachet (10) provides a second type of beneficial insect or mite, wherein preferably, the first sachet (10) on the carrier ribbon (8) provides a beneficial insect or mite in a first format and an adjacent sachet (10) provides the said beneficial insect or mite in a second format.

6. A system according to any preceding claim, wherein the carrier ribbon (8) comprises one material chosen from the range; webbing, woven material, paper, polymer, biodegradable, wherein preferably the carrier ribbon is light transmittant, comprising material having the property of allowing light to be transmitted through it.

7. A system according to any preceding claim, wherein the sachet comprises one material chosen from the range; fabric, PE coated paper, strip material.

8. A system according to any preceding claim, wherein the carrier ribbon (8) is of length in the range 80 to 90m in length.

9. A system according to any preceding claim, further comprising a drum (20) for carrying and storing the carrier ribbon (8) and sachets (10) in a rolled arrangement.

10. A system suitable for providing beneficial insects or mites in a protected cropping environment according to any of claims 1 to 9, further comprising a casing for the ribbon (8), the casing (14) having a front face and an opening (16) in the front face for deploying the ribbon (8), the carrier ribbon (8) being arranged as a roll, accommodated within the casing (14) and with a first end of the roll accessible at the opening (16).

11. A system according to claim 10, wherein a plurality of carrier ribbons (8) are housed in the casing (14), preferably wherein the number of carrier ribbons (8) in a casing (14) is five.

12. A system according to claim 10 or 11, wherein the casing (14) comprises a hexagonal container having a top face, a bottom face and six side faces, one side face including an opening (16) through which the carrier ribbon (8) is accessible and can be deployed.

13. A system according to claim 12, further comprising an attachment device for attaching the carrier ribbon to a mobile station suitable for watering or crop protection spraying for rapid deployment.

14. A method of providing beneficial insects or mites in a protected cropping environment, with a system according to any one of claims 1 to 13.

15. Use of a system according to claims 1 to 13, for the provision of beneficial insects or mites in a protected cropping environment.

## Patentansprüche

1. System, das sich zum Bereitstellen von nützlichen Insekten oder Milben in einer geschützten Ackerbau-Umgebung eignet, umfassend ein Trägerband (8) und eine Vielzahl von im Wesentlichen ebenen Päckchen (10), die auf dem Band (8) getragen sind, wobei jedes Päckchen (10) ein Ausgabefach (2) für nützliche Insekten oder Milben und ein erstes Eingrenzloch (6) auf einer Seite des Fachs und ein zweites Eingrenzloch (6) derart umfasst, dass die Insekten oder Milben emergieren können und in der geschützten Ackerbau-Umgebung ausgegeben werden, wobei die Position des zweiten Emergenzlochs (6) die Position des ersten Emergenzlochs (6) auf einer entgegengesetzten Seite des Päckchens (10) spiegelt und wobei die Päckchen (10) entlang des Trägerbandes (8) in einer regelmäßig beabstandeten Anordnung angeordnet sind, wobei die genannte Beabstandung in dem Bereich von 10 cm bis 90 cm liegt; und wobei das Trägerband (8) eine Länge in einem Bereich von 10 m bis 200 m aufweist.

2. System gemäß Anspruch 1, wobei die Päckchen (10) angeordnet sind, um vom Trägerband (8) abzuhängen und wobei die Päckchen bevorzugt in einer gleichmäßig beabstandeten Anordnung angeordnet sind.

3. System gemäß Anspruch 1 oder 2, wobei die Päckchen (10) an dem Trägerband (8) einzeln in der Reihe befestigt sind: Kleben, Schweißen, Nähen, Wärmebehandlung, Heften.

4. System gemäß irgendeinem der Ansprüche 1 bis 3, wobei das erste Emergenzloch (6) und das zweite Emergenzloch (6) in einem allerobersten Abschnitt des Fachs neben dem Trägerband (8) angeordnet sind und wobei die Emergenzlöcher (6) bevorzugt in einer Entfernung in dem Bereich von 5 bis 30 mm vom Trägerband (8) angeordnet sind.

5. System gemäß irgendeinem der voranstehenden Ansprüche, wobei ein erstes Päckchen (10) auf dem Trägerband (9) einen ersten Typ nützlicher Insekten oder Milben bereitstellt und ein angrenzendes Päckchen (10) einen zweiten Typ nützlicher Insekten oder Milben bereitstellt, wobei das erste Päckchen (10) bevorzugt auf dem Trägerband (8) ein nützliches Insekt oder eine nützliche Milbe in einem ersten Forma bereitstellt und das angrenzende Päckchen (10) das genannte nützliche Insekt oder die genannte nützliche Milbe in einem zweiten Format bereitstellt.

6. System gemäß irgendeinem der voranstehenden Ansprüche, wobei das Trägerband (8) ein Material umfasst, das aus dem Bereich ausgewählt ist: Gewebe, gewebtes Material, Papier, Polymer, biologisch abbaubares Material, wobei bevorzugt das Trägerband lichtübertragend ist, umfassend Material mit der Eigenschaft, das Licht dadurch übertragen zu lassen.

7. System gemäß irgendeinem der voranstehenden Ansprüche, wobei das Päckchen ein Material umfasst, das aus dem Bereich ausgewählt ist: Stoff, PE-beschichtetes Papier, Streifenmaterial.

8. System gemäß irgendeinem der voranstehenden Ansprüche, wobei das Trägerband (8) eine Länge im Bereich von 80 bis 90 m Länge aufweist.

9. System gemäß irgendeinem der voranstehenden Ansprüche, weiterhin umfassend eine Walze (20) zum Tragen und Speichern des Trägerbandes (8) und Päckchen (10) in einer gerollten Anordnung.

10. System, das sich zum Bereitstellen von nützlichen Insekten oder Milben in einer geschützten Ackerbau-Umgebung eignet, gemäß irgendeinem der Ansprüche 1 bis 9, weiterhin umfassend ein Gehäuse für das Band (8), wobei das Gehäuse (14) eine vordere Seite und eine Öffnung (16) in der vorderen Seite zum Abrollen des Bandes (8), wobei das Trägerband (8) als eine Rolle angeordnet ist, die innerhalb des Gehäuses (14) angeordnet ist und mit einem ersten Ende der Rolle, das an der Öffnung (16) zugänglich ist, umfasst.

11. System gemäß Anspruch 10, wobei eine Vielzahl von Trägerbändern (8) in dem Gehäuse (14) untergebracht ist, wobei bevorzugt die Anzahl von Trägerbändern (8) in einem Gehäuse (14) fünf beträgt.

12. System gemäß Anspruch 10 oder 11, wobei das Gehäuse (14) einen sechseckigen Behälter mit einer oberen Seite, einer unteren Seite und sechs seitlichen Seiten umfasst, wobei eine seitliche Seite eine Öffnung (16) einschließt, durch die das Trägerband (8) zugänglich ist und ausgerollt sein kann.

13. System gemäß Anspruch 12, weiterhin umfassend eine Befestigungsvorrichtung zum Befestigen des Trägerbandes an einer mobilen Station, die zum Bewässern oder Besprühen zum Schutz der Ernte zum raschen Ausrollen geeignet ist.

14. Verfahren zum Bereitstellen von nützlichen Insekten oder Milben in einer geschützten Ackerbau-Umgebung mit einem System gemäß irgendeinem der Ansprüche 1 bis 13.

15. Verwendung eines Systems gemäß Anspruch 1 bis 13 für die Bereitstellung von nützlichen Insekten oder Milben in einer geschützten Ackerbau-Umgebung.

## Revendications

1. Système approprié pour diffuser des insectes ou des acariens utiles dans un environnement de culture protégé, comprenant un ruban de support (8) et une pluralité de sachets sensiblement plats (10) qui sont supportés sur le ruban (8), dans lequel chaque sachet (10) comprend un compartiment de délivrance (2) destiné à contenir des insectes ou des acariens utiles et un premier trou d'émergence (6) sur un côté du compartiment et un second trou d'émergence (6) de telle sorte que des insectes ou des acariens puissent émerger et être délivrés dans l'environnement de culture protégé, dans lequel la position du second trou d'émergence (6) est la position miroir de la position du premier trou d'émergence (6) sur une face de côté opposé du sachet (10), et dans lequel les sachets (10) sont agencés le long du ruban de support (8) selon un agencement d'espacement uniforme, ledit espacement s'inscrivant à l'intérieur de la plage qui va de 10 cm à 90 cm ; et dans lequel le ruban de support (8) présente une longueur qui s'inscrit à l'intérieur de la plage qui va de 10 m à 200 m.

2. Système selon la revendication 1, dans lequel les sachets (10) sont agencés de manière à ce qu'ils dépendent du ruban de support (8), et dans lequel, de préférence, les sachets sont agencés selon un agencement d'espacement uniforme.

3. Système selon la revendication 1 ou 2, dans lequel les sachets (10) sont fixés sur le ruban de support (8) au moyen d'un moyen pris parmi la gamme qui est constituée par : le collage, le soudage, le piquage par couture, un traitement thermique, l'agrafage.

4. Système selon l'une quelconque des revendications 1 à 3, dans lequel le premier trou d'émergence (6) et le second trou d'émergence (6) sont localisés au niveau d'une partie la plus supérieure du compartiment qui est adjacente au ruban de support (8), et dans lequel, de préférence, les trous d'émergence (6) sont localisés à une distance qui s'inscrit à l'intérieur de la plage qui va de 5 mm à 30 mm par rapport au ruban de support (8).

5. Système selon l'une quelconque des revendications précédentes, dans lequel un premier sachet (10) sur le ruban de support (8) diffuse un premier type d'insectes ou d'acariens utiles et un sachet adjacent (10) diffuse un second type d'insectes ou d'acariens utiles, dans lequel, de préférence, le premier sachet (10) sur le ruban de support (8) diffuse des insectes ou des acariens utiles selon un premier format et un sachet adjacent (10) diffuse lesdits insectes ou lesdits acariens utiles selon un second format.

6. Système selon l'une quelconque des revendications précédentes, dans lequel le ruban de support (8) comprend un matériau qui est choisi au sein de la gamme qui est constituée par : une toile, un matériau tissé, du papier, un polymère, un matériau biodégradable, dans lequel, de préférence, le ruban de support laisse passer la lumière, en ce sens qu'il comprend un matériau qui a pour propriété de laisser passer la lumière par transmission au travers de lui-même.

7. Système selon l'une quelconque des revendications précédentes, dans lequel le sachet comprend un matériau qui est choisi au sein de la gamme qui est constituée par : un tissu, un papier revêtu de PE, un matériau de bande.

8. Système selon l'une quelconque des revendications précédentes, dans lequel le ruban de support (8) est d'une longueur qui s'inscrit à l'intérieur de la plage qui va de 80 m à 90 m de long.

9. Système selon l'une quelconque des revendications précédentes, comprenant en outre un tambour (20) pour supporter/transporter et stocker le ruban de support (8) et des sachets (10) selon un agencement enroulé.

10. Système approprié pour diffuser des insectes ou des acariens utiles dans un environnement de culture protégé selon l'une quelconque des revendications 1 à 9, comprenant en outre un logement pour le ruban (8), le logement (14) comportant une face avant et une ouverture (16) qui est ménagée à l'intérieur de la face avant pour déployer le ruban (8), le ruban de support (8) étant agencé en tant que rouleau, logé à l'intérieur du logement (14), une première extrémité du rouleau pouvant être accédée au niveau de l'ouverture (16) .

11. Système selon la revendication 10, dans lequel des rubans de support d'une pluralité de rubans de support (8) sont logés à l'intérieur du logement (14), de préférence dans lequel le nombre de rubans de support (8) à l'intérieur du logement (14) est de cinq.

12. Système selon la revendication 10 ou 11, dans lequel le logement (14) comprend un conteneur hexagonal qui comporte une face de sommet, une face de fond et six faces latérales, une face latérale incluant une ouverture (16) au travers de laquelle le ruban de support (8) peut être accédé et peut être déployé.

13. Système selon la revendication 12, comprenant en outre un dispositif de fixation pour fixer le ruban de support sur une station mobile qui est appropriée pour l'arrosage ou pour la pulvérisation dans un contexte de protection des cultures selon un objectif de déploiement rapide.

14. Procédé de diffusion d'insectes ou d'acariens utiles dans un environnement de culture protégé à l'aide d'un système selon l'une quelconque des revendications 1 à 13.

15. Utilisation d'un système selon les revendications 1 à 13 pour la diffusion d'insectes ou d'acariens utiles dans un environnement de culture protégé.
